# EUROPÄISCHE PATENTANMELDUNG

(11) **EP 2 857 021 A2**
(43) Veröffentlichungstag der Anmeldung: **08.04.2015**
(21) Anmeldenummer: 14200433.2
(22) Anmeldetag: 26.03.2008
(51) Int. Cl.: A61K 31/685, A61K 45/06, A61P 17/06, A61P 25/28, A61P 27/02, A61P 31/04, A61P 31/10, A61P 33/02, A61P 33/04, A61P 33/14, A61P 35/00, A61P 35/02

(54) **Oleylphosphocholin**

(30) Priorität: 26.03.2007 DE 102007014375
(62) Teilanmeldung aus: 08734781.1
(71) Anmelder: Max-Planck-Gesellschaft zur Förderung der Wissenschaften e.V., 80539 München (DE)
(72) Erfinder: Eibl, Hansjörg, 37120 Bovenden (DE)
(74) Vertreter: Dey, Michael

(57) **Zusammenfassung**

Die vorliegende Erfindung betrifft die Verwendung von Oleylphosphocholin und von Oleyl-Phospho-(N.N.-dimethyl-N-ethyl)-ethyl-ammonium zur Langzeit- und Dauerbehandlung schwerer Erkrankungen, wie z.B. Krebs, Leishmaniose, Ehrlichiose, Multiple Sklerose und Psoriasis, sowie der anderen im Text erwähnten Indikationen.

## Beschreibung

Die vorliegende Erfindung betrifft die Verwendung von Oleylphosphocholin zur Langzeit- und Dauerbehandlung schwerer Erkrankungen, wie z. B. Krebs, parasitäre Erkrankungen, bakterielle Infektionen, Pilzerkrankungen, multiple Sklerose und Psoriasis. Wichtige Grundlagen dieser Wirkung sind häufig die antiproliferativen Eigenschaften der Alkylphosphocholine, hier im Besonderen von Oleylphosphocholin.

Parasitäre Erkrankungen wie Malaria und Leishmaniose sind ein großes Problem in Ländern der dritten Welt, zunehmend aber auch in westlichen Industriestaaten. Die Ursache dafür sind zunehmender Tourismus, aber auch Gefahren, die mit der globalen Erwärmung in Verbindung gebracht werden müssen. Insbesondere die Leishmaniose - eine parasitäre Erkrankung mit oft tödlichem Ausgang - ist hier sowohl humanmedizinisch als auch veterinärmedizinisch in den Mittelpunkt des Interesses gerückt.

Wie in EP 534 445 beschrieben, ist es mit Hexadecylphosphocholin erstmals gelungen, eine wirksame orale Therapie zur Behandlung der Leishmaniose zur Verfügung zu stellen.

Hexadecylphosphocholin ist ein Vertreter aus der Substanzklasse der Alkylphosphocholine mit ausgeprägten antiproliferativen Eigenschaften. Alkylphosphocholine mit Wirkstoffqualität sind chemisch recht einfache Substanzen, nämlich Phosphocho- linester langkettiger Alkohole, die 14 bis 24 Kohlenstoffatome enthalten.

Prinzipiell können die Alkylphosphocholine mit Wirkstoffqualität gegen Leishmaniose, Krebs, Ehrlichiose und Psoriasis nach der Kettenlänge ihrer Alkylketten in zwei Gruppen eingeteilt werden. Die erste Gruppe enthält Alkylketten mit 16 bis 20 Kohlenstoffatomen, die aufgrund ihrer lytischen Eigenschaften nur oral oder topisch eingesetzt werden können. Die Nebenwirkungen bei subkutaner oder intravenöser Applikation sind Hämolyse, Zytolyse, Thrombophlebitis etc. Die Alkylphosphocholine reichern sich nach oraler Gabe in allen Körperflüssigkeiten und auch im Gewebe an, nicht aber im Organ Herz. Von großer Bedeutung ist, dass die Blut-Hirn-Schranke permeabel für Alkylphosphocholine ist. Das Gehirn nimmt relativ große Mengen an Alkylphosphocholinen auf, ohne dass sichtbare Nebenwirkungen auftreten. Je kürzer allerdings die Alkylketten sind, desto geringer wird aufgrund der erhöhten Wasserlöslichkeit die Wirksamkeit der Verbindungen.

Die zweite Gruppe umfasst Alkylketten mit 22 bis 24 Kohlenstoffatomen, die als Alkylphosphocholine nur parenteral appliziert werden können, da die Resorption dieser Substanzen aus dem Magen-Darm-Trakt gering ist (< 10 %). Auch diese Moleküle passieren die Blut-Hirn-Schranke und reichern sich in erheblichen Mengen im Gehirn an.

Überraschend ist die Sonderstellung von Hexadecylphosphocholin (C₁₆-PC) innerhalb der homologen Reihe der Alkylverbindungen C₁₄-PC, C₁₆-PC und C₁₈-PC. Nur Hexadecylphosphocholin besitzt eine verwertbar gute Antitumorwirkung, wie auch aus der Patentanmeldung EP 248 047 hervorgeht (Tierversuche in Ratten). Diese Erfahrungen wurden auch bei Protozoenerkrankungen wie z. B. der Leishmaniose bestätigt. Wieder wird die Sonderstellung von Hexadecylphosphocholin innerhalb der homologen Reihe von Alkylphosphocholinen deutlich, wie auch aus EP 534 445 hervorgeht (Tierversuche in Mäusen).

Nach den günstigen Ergebnissen mit Hexadecylphosphocholin in Tierversuchen konnten auch bei der Behandlung der humanen Leishmaniose gute Erfolge erzielt werden. Die Behandlung mit Hexadecylphosphocholin (Handelsnamen Miltefosin^{®} oder Impavido^{®}) erfolgt mit Kapseln (1.5 bis 2.5 mg Wirkstoff pro kg Körpergewicht pro Tag) über einen Zeitraum von 28 Tagen. Sehr häufige Nebenwirkungen sind Erbrechen, Durchfall, Übelkeit, Anstieg der Leberenzyme und Erhöhung von Kreatinin. Immungeschwächte Patienten sollen nicht behandelt werden.

Die unangenehmen Nebenwirkungen mit Hexadecylphosphocholin wurden bei der Behandlung der caninen Leishmaniose besonders deutlich. Canine Leishmaniose ist durch den Import von Hunden aus südlichen Ländern ein zunehmendes Problem. Die Wirksamkeit von Hexadecylphosphocholin gegen canine Leishmaniose setzte erst mit 3 mg pro kg KGW pro Tag ein. Höhere Dosen wurden nicht vertragen, geringere Dosen waren kaum wirksam. Damit war eine praktische Anwendung von Hexadecylphosphocholin in der Tiergesundheit zur Behandlung der caninen Leishmaniose nicht effektiv infolge unerfreulicher Nebenwirkungen wie Übelkeit, Erbrechen und einer starken Einschränkung der Nierenfunktion unter den Bedingungen der Therapie. Außerdem problematisch waren hämolytische und zytolytische Eigenschaften, wenn von einer oralen Applikation abgegangen und subkutane oder intravenöse Gaben angewendet wurden.

Mit Hilfe von liposomalen Formulierungen konnten dann die genannten Schwierigkeiten überwunden werden. Durch den Einbau von Hexadecylphosphocholin in liposomale Hüllen, die mit dem Zusatz von Cholesterin erreicht wurden, konnten die hämolytischen und zytolytischen Eigenschaften unterdrückt werden. Die therapeutische Wirkung von Hexadecylphosphocholin wurde durch den Einbau in Liposomen kaum verbessert. Allerdings waren die Nebenwirkungen deutlich reduziert.

Eine entscheidende Verbesserung wurde erst durch chemische Strukturvariation erzielt. Bei einem Vergleich der therapeutischen Wirkung von drei Alkylphosphocholinen gleicher Kettenlänge im Tierversuch - C₁₈-PC (Octadecyl), C_{18:1(trans)}-PC (Elaidyl) und C_{18:1(cis)}-PC (Oleyl) - erwies sich Oleylphosphocholin den anderen Alkylphosphocholinen gleicher Kettenlänge deutlich überlegen. Insbesondere fällt auf, dass die geringe Toxizität im Tierversuch zu einer großen therapeutischen Breite führt. Nach früheren Erfahrungen mit Hexadecylphosphocholin und dem dort beobachteten günstigen Effekt von liposomalen Formulierungen auf die Verträglichkeit wurde Oleylphosphocholin ebenfalls in liposomaler Form eingesetzt.

Von den unterschiedlichen liposomalen Formulierungen mit Oleylphosphocholin ist DE 10 2004 055 284 besonders zu erwähnen. Diese Formulierung besteht aus einer einfachen Lipidkomposition-Oleylphosphocholin, Cholesterin und Ölsäure - in einer liposomalen Formulierung, die steril filtriert, hitzesterilisiert und bei 4 bis 8 °C problemlos 3 Jahre gelagert werden kann. Diese Oleylphosphocholin-Liposomen können einfach hergestellt werden. Sie sind eine wichtige Arzneiform, um sehr hartnäckige, chronische Krankheiten zu behandeln, bei denen möglicherweise eine orale Gabe nicht sinnvoll ist wegen Störungen des Resorptionsverhaltens im Magen-Darm-Trakt.

Der Kostendruck in unserem Gesundheitswesen erfordert zwingend, Wirkstoffe und Behandlungsformen zu entwickeln, die schwere Erkrankungen heilen oder kontrollieren können. Nach Möglichkeit sollen diese Arzneimittel dem Patienten und unserem Gesundheitssystem kostengünstig zur Verfügung gestellt werden. Unter diesen Gesichtspunkten ist eine liposomale Formulierung immer von Nachteil.

Aufgabe der vorliegenden Erfindung ist es deshalb, einerseits ein Arzneimittel bereitzustellen, welches einfach herzustellen und kostengünstig ist, das aber andererseits die Behandlung schwerer Erkrankungen ermöglicht.

Diese Aufgabe wird erfindungsgemäß gelöst durch die Verwendung von Verbindungen der Formel (I)

R¹-O-PO₂⁽⁻⁾-O-(CH₂)₂-N⁽⁺⁾-R²(CH₃)₂

worin
R¹ ein Oleylrest ist und
R² CH₃ oder CH₂CH₃ bedeutet,
zur Herstellung eines Arzneimittels zur Behandlung von parasitären Erkrankungen, wie insbesondere von Leishmaniose oder Malaria, Krebs, multiple Sklerose, Psorias, bakteriellen Infektionen, wie z.B. Ehrlichiose, und/oder Pllzerkrankungen ist.

Überraschenderweise wurde festgestellt, dass Verbindungen der Formel (I) und insbesondere Oleylphosphocholin einerseits eine verringerte Toxizität aufweisen, andererseits in sehr geringen Dosierungen bereits wirksam sind. Dies ermöglicht es, diese Wirkstoffe als Arzneimittel einzusetzen, ohne dass unerwünschte Nebenwirkungen beobachtet werden. Weiterhin sind besondere Formulierungen, beispielsweise Liposomenformulierungen, nicht mehr erforderlich. Bevorzugt werden die Verbindungen deshalb erfindungsgemäß in einer nicht-liposomalen Form eingesetzt. Vielmehr ist eine einfache orale Verabreichung des Wirkstoffs möglich und aufgrund der geringen benötigten Dosierungen tritt kein Erbrechen und keine Übelkeit bei den Patienten auf. Weiterhin ist auch eine subkutane Verabreichung, wiederum aufgrund der geringen erforderlichen Wirkstoffmenge, ohne Reizung möglich. Aufgrund der hohen Wirksamkeit und der dadurch benötigten geringen Wirkmenge sind auch Langzeit- oder/und Dauerbehandlungen möglich, insbesondere Behandlungen über eine Dauer von mehr als 2 Wochen, insbesondere von mehr als 4 Wochen, aber auch von mehr als 2 Monaten, insbesondere von mehr als 6 Monaten.

Nach den günstigen Erfahrungen mit Oleylphosphocholin in Liposomen in Verbindung mit der dort beobachteten großen therapeutischen Breite haben wir überraschenderweise festgestellt, dass Oleylphosphocholin in therapeutisch effektiven Dosen auch ohne liposomale Verpackung eingesetzt werden kann: In allen Applikationsformen von oral über subkutan zu intravenös. Überraschenderweise treten in therapeutisch effektiven Dosen die bei Hexadecylphosphocholin beobachteten Nebenwirkungen nicht auf. Selbst bei Langzeitanwendungen über mehrere Monate wird keine Verschlechterung der Nierenwerte (Kreatinin-Anstieg) beobachtet - ganz im Gegenteil: Die Nierenwerte normalisieren sich sehr rasch unter der Therapie. Diese Ergebnisse bestätigen die überragende Sonderstellung von Oleylphosphocholin in der Reihe der Alkylphosphocholine. Oleylphoshocholin ist das erste Alkylphosphocholin das oral und intravenös eingesetzt werden kann.

Von großer Bedeutung für eine praktische Anwendung ist weiterhin, dass die Stabilität von Oleylphosphocholin in Injektions- oder Infusionslösungen oder auch in Form von Tabletten selbst bei 30 °C über einen längeren Zeitraum gewährleistet werden kann. Für den Einsatz in der Tiermedizin bietet sich weiter an, dass Mischungen aus Oleylphosphocholin (90 %) und Hexadecylphosphocholin (10 %) eingesetzt und extrem kostengünstig aus Olivenöl zugänglich gemacht werden können.

Erfindungsgemäß wurde Hexadecylphosphocholin durch chemische Strukturvariation verbessert und insbesondere die Toxizität verringert. Das ist mit der Verlängerung der Alkylkette um zwei CH₂-Gruppen und der Verwendung einer cis-Doppelbindung hervorragend gelungen. Das Produkt ist Oleylphosphocholin. Aus der Gruppe der Alkylphosphocholine ist dies ein Molekül mit besonderen und herausragenden therapeutischen Eigenschaften. Besonders bevorzugt wird erfindungsgemäß deshalb Oleylphosphocholin eingesetzt.

Weiterhin wurde erfindungsgemäß festgestellt, dass Verbindungen mit einer zusätzlichen CH₂-Gruppe am Stickstoff (also N.N-Dimethyl-N-ethyl-ethanolamin-Verbindungen) im Vergleich zu Cholinverbindungen einen Zuwachs an therapeutischer Breite aufweisen. Deshalb ist zusätzlich zu Oleylphoshocholin auch noch Oleyl-Phospo-(N.N.-dimethyl-N-ethyl)-ethylammonium besonders zu erwähnen. Die zusätzliche CH₂-Gruppe am Stickstoff führt zu einem Zuwachs an therapeutischer Breite von etwa 25% im Vergleich zu Oleylphosphocholin.

Überraschenderweise wurde gefunden, dass Oleylphosphocholin bereits in sehr geringen Mengen wirkt und deshalb auch in physiologischer Kochsalzlösung subkutan, intravenös und intramuskulär ohne Nebenwirkungen appliziert werden konnte. Eine Formulierung als Liposom ist nicht erforderlich. Auch die orale und topische Gabe ist möglich. Die frühere Erfahrung, dass Alkylphosphocholine mit 16 bis 20 Kohlenstoffatomen wegen heftiger Nebenwirkungen wie Hämolyse, Zytolyse und Thrombophlebitis nicht in freier Form als mizellare Lösung eingesetzt werden können, trifft überraschenderweise für Oleylphosphocholin nicht zu. Der Grund dafür ist, dass Oleylphosphocholin bei Hund und Mensch in sehr geringen täglichen Dosen von z.B. 0,15 bis 1,5 mg/kg KGW (Körpergewicht) gut wirkt, während Hexadecylphosphocholin in diesen Dosierungen gegen Leishmaniose ohne Wirkung bleibt. Es ist deshalb eine direkte Verwendung des freien Wirkstoffs möglich. In dem erfindungsgemäß hergestellten Arzneimittel liegt der Wirkstoff insbesondere nicht in liposomaler Form vor.

Die erfindungsgemäßen Verbindungen sind bereits in sehr geringen Dosierungen wirksam. Typische Dosierungseinheiten betragen von 0,1 bis 5 mg, insbesondere bis 4 mg, insbesondere von 0,2 bis 2 mg und noch mehr bevorzugt von 0,3 bis 0,9 mg pro KGW pro Tag. Die Behandlung kann also mit äußerst geringen Mengen durchgeführt werden: Tagesdosen von 0,1 bis 0,9 mg/kg KGW reichen aus.

Die geringen Dosen öffnen ein weites Fenster für neue Anwendungen, insbesondere für Langzeitapplikationen oder/und Dauerapplikationen. Das war bisher mit Hexadecylphosphocholin nicht möglich aufgrund der genannten Nebenwirkungen, insbesondere der Nierentoxizität.

Bevorzugt sind Tagesdosen pro Kilogramm Körpergewicht des Patienten (wobei der Patient ein Mensch oder ein Tier sein kann) von 0,1 bis 2 mg, insbesondere von 0,15 bis 1,5 mg, bevorzugt von 0,2 bis 1,2 mg, mehr bevorzugt von 0,3 bis 0,9 mg und noch mehr bevorzugt von 0,5 bis 0,8 mg.

Geeignete Darreichungsformen sind beispielsweise übliche physiologische Lösungen, insbesondere physiologische Kochsalzlösung, Tabletten oder Aerosole.

Erfindungsgemäß werden die Verbindungen der Formel (I) bevorzugt zur Behandlung von Säugetieren und am meisten bevorzugt zur Behandlung von Hunden oder Menschen eingesetzt.

Die Wirkstoffe der Formel (I) sind insbesondere geeignet, um lebensbedrohende Krankheiten wie Leishmaniose zu heilen und schwere Erkrankungen wie Krebs langfristig zu kontrollieren. Gerade unsere Erfahrung mit der Langzeit- oder sogar Dauerbehandlung von Hunden mit Oleylphosphocholin haben gezeigt, dass diese Tiere langfristig profitieren und in ausgezeichnetem Gesundheitszustand das normale Lebensende erreichen können.

Erfindungsgemäß können Verbindungen der Formel (I) insbesondere zur Behandlung oder/und Prophylaxe von Protozoenerkrankungen eingesetzt werden. Sie weisen hervorragende Wirksamkeiten gegen Protozoen und davon hervorgerufene Erkrankungen auf. Sie sind insbesondere wirksam gegen Plasmodien und damit geeignet für die Behandlung oder Prophylaxe von Schlafkrankheit, gegen Amöben, z. B. Endamöben und Acanthamöben, für die Behandlung oder Prophylaxe von Amöbenruhr und Encephalitis und insbesondere gegen Leishmaniose. Neben der Behandlung von Menschen können erfindungsgemäß die Arzneimittelzubereitungen vorteilhaft auch bei der Behandlung von Tieren und insbesondere bei Leishmaniose bei Hunden eingesetzt werden. Besonders bevorzugt werden erfindungsgemäß die Verbindungen der Formel (I) zur Behandlung von Leishmaniose oder/und zur Behandlung von durch Amöben hervorgerufene Erkrankungen eingesetzt.

Weiterhin hat sich gezeigt, dass die Verbindungen der Formel (I) hervorragende Antitumormittel darstellen. Sie können somit zur Behandlung oder/und Prophylaxe von Krebs, insbesondere von Leukämie und soliden Tumoren eingesetzt werden. Auch bei der Behandlung von Krebs in Hunden haben sich die Substanzen durchaus bewährt. So konnten z. B. Morbus Cushin und Blasentumoren mit gutem Erfolg behandelt werden.

Darüber hinaus können sie zur Stimulierung der Leukopoese sowie zur Behandlung von Erkrankungen, die durch Arthropoden verursacht werden und von Akarinosis eingesetzt werden.

Überraschenderweise hat sich herausgestellt, dass die Verbindungen der Formel (I) auch eine gute Wirksamkeit gegen Akarinosis, insbesondere Räude und gegen durch Arthropoden sowie durch Ascariden, wie etwa Milben oder Zecken verursachte Erkrankungen aufweist.

Weiterhin zeigen die Verbindungen der Formel (I) auch eine hervorragende Wirksamkeit gegenüber bakteriellen Erkrankungen. Sie können deshalb auch zur Behandlung oder/und Prophylaxe von bakteriellen Erkrankungen, insbesondere zu Behandlung oder/und Prophylaxe von Ehrlichiose eingesetzt werden. Ehrlichiose ist eine bakterielle Erkrankung, die durch Zecken übertragen wird. Bei der Behandlung von Hunden wurde nicht nur eine deutliche Reduzierung des Ehrlichiosetiters festgestellt, sondern Heilung erreicht.

Auch Augenerkrankungen, die mit zellulären Proliferationen einhergehen wie z. B. die proliferative Vitreoretinopathie oder Netzhautablösungen am Auge, die operativ nur schwer zu behandeln sind, und ebenfalls oftmals mit sehr stark proliferierenden Zellen einhergehen, können erfolgreich behandelt bzw. vermieden werden.

Die Behandlung der genannten Erkrankungen kann praktisch nebenwirkungsfrei durchgeführt werden. Überraschenderweise führt der Einsatz der Verbindungen der Formel (I) nicht zu der bei Chemotherapien gefürchteten Immunsuppression, sondern sogar zu einer Stimulation der Leukopoese. Mit zunehmender Dauer der Therapie normalisiert sich auch das Blutbild. Mit anderen Worten, die hier beschriebenen Verbindungen der Formel (I) als Wirkstoff sind für Langzeittherapien sehr geeignet und ermöglichen bei therapeutisch effektiven Dosen eine nebenwirkungsfreie Behandlung.

Die hierin besonders hervorgehobenen Verbindungen der Formel (I) sind solche mit mindestens einer cis-Doppelbindung im Molekül, besonders bevorzugt Oleylphosphocholin. Alkylphosphocholine mit cis-Doppelbindungen zeichnen sich durch eine wesentlich größere therapeutische Breite aus, d. h., es können deutlich höhere Dosen appliziert werden als bei gesättigten Alkylphosphocholinen. Das ist dann besonders vorteilhaft, wenn die Therapie mit Strahlentherapie kombiniert wird. Mit Oleylphosphocholin dotierte Tumorzellen sind gegenüber Strahlentherapie sensibilisiert und besonders anfällig.

Es wurde überraschenderweise festgestellt, dass die Verbindungen der Formel (I) auch zur Behandlung oder/und Prophylaxe an Tieren, insbesondere bei Hunden hervorragend geeignet sind. Entsprechend werden die Verbindungen der Formel (I) bevorzugt in der Veterinärmedizin und dort insbesondere zur Behandlung von Tumor- und Protozoenerkrankungen eingesetzt. Insbesondere bei Hunden, bei denen bisherige, für den Menschen geeignete Mittel oftmals versagt haben, konnten dabei hervorragende Ergebnisse erzielt werden. So können beispielsweise Leishmaniosen und Ehrlichiosen bei Tieren und insbesondere bei Hunden mit den Verbindungen der Formel (I) erfolgreich behandelt werden. Besonders bevorzugt wird hierbei Oleylphosphocholin als Wirkstoff eingesetzt.

Weiterhin wurde festgestellt, dass mit den Verbindungen der Formel (I) auch Augenerkrankungen erfolgreich behandelt werden können, insbesondere Augenerkrankungen, die mit unkontrollierten zellulären Prozessen einhergehen.

Die Verbindungen der Formel (I), insbesondere in freier Form (also nicht als Liposomen) sind somit besonders geeignet zur Behandlung oder/und Prophylaxe von Krebs, von Protozoenerkrankungen wie Leishmaniosen und Amöbenerkrankungen, von Acariasis und von Erkrankungen, die durch Arthropoden verursacht werden sowie von bakteriellen Erkrankungen, wie z. B. Ehrlichiose, geeignet. Auch Augenerkrankungen, die mit unkontrollierten zellulären Prozessen einhergehen, können günstig beeinflusst werden.

Die Verbindungen der Formel (I) haben auch eine ausgeprägte Wirkung gegen Pilzerkrankungen, insbseondere gegen systemische Pilzerkrankungen. Die Verbindungen können deshalb zusätzlich auch im Pflanzenschutz eingesetzt werden.

Die Möglichkeit der Langzeit- oder Dauerbehandlung erschließt ganz neue Therapiefelder. Dadurch können auch chronische Erkrankungen wie Multiple Sklerose dauerhaft positiv beeinflusst und das Fortschreiten der Erkrankung gehemmt werden.

Weiterhin interessante Anwendungsmöglichkeiten finden sich bei Erkrankungen, die mit allergischen Reaktionen einhergehen, ebenso wie Erkrankungen, die Entzündungsreaktionen beinhalten. Dabei ist der gemeinsame Hintergrund immer, dass die Verbindungen der Formel (I) das unkontrollierte Zellwachstum, die Zellproliferation hemmen.

Mit den jetzt zur Verfügung stehenden, wenig toxischen Verbindungen der Formel (I) können frühere Erfahrungen bei der Behandlung von Psoriasis wieder aktualisiert werden. Die früher schon erzielten günstigen Ergebnisse sollten mit Oleylphosphocholin noch deutlich verbessert werden.

### BEISPIELE

### Beispiel 1

Im Folgenden werden Dosierungsbereiche angegeben für Hexadecylphosphocholin sowie für Oleylphosphocholin, wobei die erste Zahl beginnende Wirkung und die zweite Zahl beginnende Toxizität anzeigt (siehe Tabelle 1). Hexadecylphosphocholin ist ein zugelassenes Arzneimittel, Oleylphosphocholin ist eine Verbindung der Formel (I) gemäß vorliegender Erfindung. Für die Oleylphosphocholin Anwendung im Menschen können deshalb zunächst nur geschätzte Werte angegeben werden, die entsprechend mit "g" indiziert sind (siehe Tabelle 1).

**Tabelle 1**

| HePC (MG 407.57) | |
|---|---|
| Ratte | 20 - 70 µmol = 8.0 - 30.0 mg |
| Hund | 7 µmol = 3.0 mg |
| Mensch | 4 - 6 µmol = 1.5 - 2.5 mg |
| | |

| OIPC (MG 433.61) | |
|---|---|
| Ratte | 10 - 150 µmol = 4.0 - 65.0 mg |
| Hund | 0.6 - 10 µmol = 0.3 - 4.5 mg |
| Mensch | 0.6 - 10 µmol = 0.3 - 4.5 mg (Werte geschätzt) |

### Beispiel 2

Die Herstellung der Verbindungen der Formel (I) analog der in DE 4132344 A1 beschriebenen Vorgehensweise erfolgen.

## Patentansprüche

1. Verwendung der Verbindungen der Formel (I)
R¹-O-PO₂⁻-O-(CH₂)₂-N⁺R²(CH₃)₂ (I)
worin
R¹ ein Oleylrest ist und
R²CH₃ oder CH₂CH₃ bedeutet,
zur Herstellung eines Arzneimittels zur Behandlung von Protozoenerkrankungen, insbesondere Leishmaniose, Schlafkrankheit, Amöbenruhr oder Encephalitis, von bakteriellen Erkrankungen, insbesondere Ehrlichiose, von Krebs, insbesondere Leukämie, soliden Tumoren, Morbus Cushin oder Blasentumoren, von Multipler Sklerose, von Akarinosis, von Augenerkrankungen, von Pilzerkrankungen und/oder von Psoriasis.

2. Verwendung nach Anspruch 1, **dadurch gekennzeichnet, dass** das Arzneimittel zur Verabreichung in einer Menge von 0,1 bis 1,2 mg/kg Körpergewicht/Tag vorgesehen ist.

3. Verwendung nach Anspruch 1 oder 2, **dadurch gekennzeichnet, dass** das Arzneimittel zur Verabreichung in einer Menge von 0,1 bis 0,9 mg/kg Körpergewicht/Tag vorgesehen ist.

4. Verwendung nach einem der vorhergehenden Ansprüche, **dadurch gekennzeichnet, dass** das Arzneimittel zur Verabreichung in einer Menge von 0,5 bis 0,8 mg/kg Körpergewicht/Tag vorgesehen ist.

5. Verwendung nach einem der vorhergehenden Ansprüche, **dadurch gekennzeichnet, dass** die Verbindung der Formel (I) in freier, nicht-liposomaler Form vorliegt.

6. Verwendung nach einem der vorhergehenden Ansprüche, **dadurch gekennzeichnet, dass** der Wirkstoff in physiologischer Lösung oder in Tabletten vorliegt.

7. Verwendung nach einem der vorhergehenden Ansprüche, **dadurch gekennzeichnet, dass** das Arzneimittel zur oralen oder subkutanen Verabreichung vorgesehen ist.

8. Verwendung nach einem der vorhergehenden Ansprüche, **dadurch gekennzeichnet, dass** es sich bei der Verbindung der Formel (I) um Oleylphosphocholin handelt.

9. Verwendung nach einem der vorhergehenden Ansprüche, **dadurch gekennzeichnet, dass** weitere Wirkstoffe zur Herstellung des Arzneimittels verwendet werden.

10. Verbindung der Formel (I)
R¹-O-PO₂⁻-O(CH₂)₂-N⁺R²(CH₃)₂ (I)
worin
R¹ ein Oleylrest ist und
R² CH₃ oder CH₂CH₃ bedeutet,
zur Verwendung zur Behandlung von Protozoenerkrankungen, insbesondere Leishmaniose, Schlafkrankheit, Amöbenruhr oder Encephalitis, von bakteriellen Erkrankungen, insbesondere Ehrlichiose, von Krebs, insbesondere Leukämie, soliden Tumoren, Morbus Cushin oder Blasentumoren, von Multipler Sklerose, von Akarinosis, von Augenerkrankungen, von Pilzerkrankungen und/oder von Psoriasis.

11. Verbindung der Formel (I) zur Verwendung nach Anspruch 10, **dadurch gekennzeichnet, dass** sie in einer Menge von 0,1 bis 1,2 mg/kg Körpergewicht/Tag, insbesondere in einer Menge von 0,1 bis 0,9 mg/kg Körpergewicht/Tag und noch mehr bevorzugt in einer Menge von 0,5 bis 0,8 mg/kg Körpergewicht/Tag verabreicht wird.

12. Verbindung der Formel (I) zur Verwendung nach einem der Ansprüche10 oder 11, **dadurch gekennzeichnet, dass** die Verbindung der Formel (I) in freier, nicht-liposomaler Form vorliegt.

13. Verbindung der Formel (I) zur Verwendung nach einem der Ansprüche 10 bis 12, **dadurch gekennzeichnet, dass** der Wirkstoff in physiologischer Lösung oder in Tabletten vorliegt.

14. Verbindung der Formel (I) zur Verwendung nach einem der Ansprüche 10 bis 13, **dadurch gekennzeichnet, dass** das Arzneimittel zur oralen oder subkutanen Verabreichung vorgesehen ist.

15. Verbindung der Formel (I) zur Verwendung nach einem der Ansprüche 10 bis 14, **dadurch gekennzeichnet, dass** es sich bei der Verbindung der Formel (I) um Oleylphosphocholin handelt.
